(19) Europäisches Patentamt / European Patent Office / Office européen des brevets

(11) EP 2 688 929 B1

(12) **EUROPÄISCHE PATENTSCHRIFT**

(45) Veröffentlichungstag und Bekanntmachung des Hinweises auf die Patenterteilung:
**15.01.2020 Patentblatt 2020/03**

(21) Anmeldenummer: **12709337.5**

(22) Anmeldetag: **21.03.2012**

(51) Int Cl.:
***C08G 18/08*** *(2006.01)* ***C08G 18/10*** *(2006.01)*
***C08G 18/42*** *(2006.01)* ***C08G 18/75*** *(2006.01)*
***A61K 8/87*** *(2006.01)* ***A61Q 5/06*** *(2006.01)*

(86) Internationale Anmeldenummer:
**PCT/EP2012/054976**

(87) Internationale Veröffentlichungsnummer:
**WO 2012/130683 (04.10.2012 Gazette 2012/40)**

(54) **POLYURETHANHARNSTOFF-MISCHUNG FÜR DIE HAARKOSMETIK**

POLYURETHANE UREA MIXTURE FOR HAIR COSMETICS

MÉLANGE DE POLYURÉTHANE-URÉE POUR LA COSMÉTIQUE CAPILLAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.03.2011 EP 11159697**

(43) Veröffentlichungstag der Anmeldung:
**29.01.2014 Patentblatt 2014/05**

(73) Patentinhaber: **Covestro Deutschland AG**
**51373 Leverkusen (DE)**

(72) Erfinder:
- **VIALA, Sophie**
  **50935 Köln (DE)**
- **DÖRR, Sebastian**
  **40593 Düsseldorf (DE)**

(74) Vertreter: **Levpat**
**c/o Covestro AG**
**Gebäude 4825**
**51365 Leverkusen (DE)**

(56) Entgegenhaltungen:
**EP-A2- 1 923 411**
**WO-A1-2007/115697**
**WO-A1-2009/118105**
**WO-A1-2010/118948**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 688 929 B1

## Beschreibung

Die vorliegende Erfindung betrifft einen speziellen Polyurethanharnstoff, der insbesondere in der Form einer Mischung mit üblichen Lösungsmitteln im Bereich der Haarkosmetik eingesetzt werden kann. Weitere Gegenstände der Erfindung sind die Verwendung des Polyurethan-Harnstoffs in der Kosmetik, eine kosmetische Zusammensetzung enthaltend den Polyurethan-Harnstoff, die Verwendung der kosmetischen Zusammensetzung in der Kosmetik und ein Verfahren zum Formen von Frisuren unter Verwendung der kosmetischen Zusammensetzung.

Zur Gestaltung und Stabilisierung vielseitiger Frisuren werden Produkte eingesetzt, die als Haarfestiger bekannt sind. Haarfestiger gibt es meistens in Form von Schaumfestigern oder Haarsprays, wobei sie sich in ihrer Zusammensetzung kaum unterscheiden. Schaumfestiger werden auf das feuchte Haar als Hilfsmittel zur Modellierung der Frisur aufgetragen. Im Gegensatz hierzu, werden Haarsprays auf trockene fertig gestylte Haare zur Fixierung der Frisur aufgebracht.

In dem Falle von Haarsprays und Schaumfestigern liegen die Mittel zur Fixierung oder Gestaltung der Frisur üblicherweise als aus Aerosolbehältern, Quetschflaschen oder durch eine Pump-, Sprüh- oder Schäumvorrichtungen versprühbare Präparate vor, die aus einer alkoholischen oder wäßrigalkoholischen Lösung von filmbildenden natürlichen oder synthetischen Polymeren bestehen. Diese Polymere können aus der Gruppe der nichtionischen, kationischen, amphoteren oder anionischen Polymeren ausgewählt werden.

Als filmbildende Polymere werden im Stand der Technik häufig anionische oder amphotere Polymere auf Basis von Acrylaten eingesetzt. Bekannt ist aber auch die Verwendung von Polyurethanen und Polyurethanharnstoffen als Filmbildner. So sind beispielsweise in der WO 2009/118105 A1 Haarfestiger Zusammensetzungen beschrieben, die einen Polyurethanharnstoff erhalten, der durch Umsetzung eines wasserunlöslichen nicht wasserdispergierbaren isocyanatfunktionellen Polyurethanprepolymers mit einer aminofunktionellen Verbindung erhältlich ist. Die hier offenbarten Haarfestiger Zusammensetzungen sind gut zur Stabilisierung von Frisuren geeignet. Allerdings können aus den bekannten wasserbasierten Polyurethanharnstoffen keine transparenten Mischungen hergestellt werden. So sind Systeme, die neben den Polyurethanharnstoffen die im Bereich der Haarkosmetik gebräuchlichen Lösungsmittel wie Wasser und Ethanol enthalten, trüb. Dies wird für viele Anwendungen als nachteilig empfunden.

Aufgabe der vorliegenden Erfindung war es daher einen Polyurethanharnstoff bereit zu stellen, der die vorteilhaften Fixierungseigenschaften der aus der WO 2009/118105 A1 bekannten Systeme aufweist und von dem darüber hinaus in im Bereich der Kosmetik üblichen Lösungsmitteln klare Mischungen hergestellt werden können.

Diese Aufgabe ist erfindungsgemäß durch einen Polyurethanharnstoff gelöst, der durch Umsetzung

a) einer einzigen Polyisocyanat-Komponente,

b) wenigstens einer polymeren Polyolkomponente,

c) wenigstens einer hydrophilierenden Komponente und

d) einer einzigen aminofunktionellen Kettenverlängerer-Komponente,

wobei die Polyisocyanat-Komponente a) > 75 mol% Isophorondiisocyanat (IPDI) und wenigstens eine polymere Polyolkomponente b) einen Polyester umfasst, und die hydrophilierende Komponente c) ein Sulfonat ist, und die aminofunktionelle Kettenverlängerer-Komponente d) > 75 mol % Isophorondiamin (IPDA) umfasst, erhältlich ist.

Erfindungsgemäß wird unter einer aminofunktionellen Kettenverlängerer-Komponente eine Komponente verstanden, die wenigstens eine Verbindung mit zwei isocyanatreaktiven Aminogruppen und keinen hydrophilierenden Gruppen umfasst.

Gemäß einer ersten bevorzugten Ausführungsform des erfindungsgemäßen Polyurethanharnstoffs kann die Polyisocyanat-Komponente a) ≥ 80 mol%, bevorzugt ≥ 85 mol%, weiter bevorzugt 95 mol% und besonders bevorzugt 100 mol% IPDI umfassen.

Weitere - zusätzlich zu IPDI in einem molaren Anteil von weniger als 25 mol% einsetzbare - Polyisocyanate der Komponente a) sind die dem Fachmann an sich bekannten aromatischen, araliphatischen, aliphatischen oder cycloaliphatischen Polyisocyanate einer NCO-Funktionalität von ≥ 2.

Beispiele solcher Polyisocyanate sind 1,4-Butylendiisocyanat, 1,6-Hexamethylendiisocyanat (HDI), 2,2,4 und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3- und/oder 1,4-Bis-(2-isocyanato-prop-2-yl)-benzol (TMXDI), 1,3-Bis(isocyanatomethyl)benzol (XDI), Alkyl-2,6-diisocyanatohexanoat (Lysindiisocyanate) mit C1-C8-Alkylgruppen, sowie 4-Isocyanatomethyl-1,8-octandiisocyanat (Nonantriisocyanat) und Triphenylmethan-4,4',4"-triisocyanat.

Neben den vorstehend genannten Polyisocyanaten können anteilig auch modifizierte Diisocyanate oder Triisocyanate mit Uretdion-, Isocyanurat-, Urethan-, Allophanat-, Biuret-, Iminooxadiazindion- und/oder Oxadiazintrionstruktur mit eingesetzt werden.

Bevorzugt handelt es sich um Polyisocyanate oder Polyisocyanatgemische der vorstehend genannten Art mit ausschließlich aliphatisch und / oder cycloaliphatisch gebundenen Isocyanatgruppen und einer mittleren NCO-Funktionalität der Mischung von 2 bis 4, bevorzugt von 2 bis 2,6 und besonders bevorzugt von 2 bis 2,4.

Besonders bevorzugt werden - falls neben IPDI weitere Polyisoyanate in der Komponente a) eingesetzt werden 1,6-Hexamethylendiisocyanat, die isomeren Bis(4,4'-isocyanatocyclohexyl)methane, sowie deren Mischungen verwendet.

Bevorzugt ist ebenfalls, wenn die polymere Polyolkomponente b) ein zahlenmittlere Molekulargewichte von $\geq$ 400 und $\leq$ 8000 g/mol, besonders bevorzugt von 600 bis 3000 g/mol eingesetzt und / oder eine mittlere OH-Funktionalitäten von 1,5 bis 6,. bevorzugt von 1,8 bis 3 und besonders bevorzugt von 1,9 bis 2,1 aufweist.

Erfindungsgemäß ist, wenn die polymere Polyolkomponente b) einen Polyester, bevorzugt einen Polyester basierend auf Adipinsäure umfasst oder daraus besteht.

Mögliche Bestandteile der polymeren Polyolkomponente b) sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in b) einzeln oder in beliebigen Mischungen untereinander eingesetzt werden.

Polyesterpolyole sind beispielsweise die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele hierfür geeigneter Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Neopentylglykol und Hydroxypivalinsäureneopentylglykolester bevorzugt sind. Daneben können auch Polyole wie Trimethylolpropan, Glycerin, Erythrit, Pentaerythrit, Trimethylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols größer als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure, ganz besonders bevorzugt ist Adipinsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

In der Komponente b) können auch Hydroxylgruppen aufweisende Polycarbonate, bevorzugt Polycarbonatdiole, mit zahlenmittleren Molekulargewichten Mn von 400 bis 8000 g/mol, bevorzugt von 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art. Die Hydroxylgruppen aufweisenden Polycarbonate sind bevorzugt linear gebaut.

Ebenfalls können in der Komponente b) Polyetherpolyole eingesetzt werden. Geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxide und/oder Epichlorhydrin an di- oder polyfunktionelle Startermoleküle.

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol. Bevorzugte Startermoleküle sind Wasser, Ethylenglykol, Propylenglykol,

1,4-Butandiol, Diethylenglykol und Butyldiglykol.

Für den erfindungsgemäßen Polyurethanharnstoff ist vorgesehen, dass die hydrophilierende Komponente c) ein Sulfonat ist.

Geeignete anionisch bzw. potentiell anionisch hydrophilierende Verbindungen der Komponente c) sind Verbindungen, die mindestens eine isocyanatreaktive Gruppe wie eine Hydroxylgruppe oder Aminogruppe aufweisen sowie mindestens eine Funktionalität wie z.B. $-COO^-M^+$, $-SO_3^-M^+$,$-PO(O^-M^+)_2$ mit $M^+$ beispielsweise gleich Metallkation, $H^+$, $NH4^+$, $NHR_3^+$, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann. Geeignete anionisch oder potentiell anionisch hydrophilierende Verbindungen sind Mono- und Dihydroxycarbonsäuren, Mono- und Dihydroxysulfonsäuren, sowie Mono- und Dihydroxyphosphonsäuren und ihre Salze. Beispiele solcher anionischen bzw. potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und $NaHSO_3$, wie es in DE-A 2 446 440, Seite 5 - 9, Formel I-III beschrieben ist. Bevorzugte anionische oder potentiell anionische Hydrophilierungsmittel der Komponente c) sind solche der vorstehend genannten Art, die über Carboxylat- bzw. Carbonsäuregruppen und/oder Sulfonatgruppen verfügen.

Geeignete nichtionisch hydrophilierende Verbindungen der Komponente c) sind z.B. Polyoxyalkylenether, die mindestens eine Hydroxy- oder Aminogruppe, bevorzugt mindestens eine Hydroxygruppe enthalten.

Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38 beschrieben). Diese Verbindungen sind entweder reine Polyethylenoxidether oder gemischte Polyalkylenoxidether, wobei sie dann aber mindestens 30 mol-%, bevorzugt mindestens 40 mol-%, bezogen auf alle enthaltenen Alkylenoxideinheiten an Ethylenoxideinheiten, enthalten. Besonders bevorzugte nichtionische Verbindungen sind monofunktionelle, gemischte Polyalkylenoxidpolyether, die 40 bis 100 mol-% Ethylenoxid- und 0 bis 60 mol-% Propylenoxideinheiten aufweisen.

Zur Hydrophilierung können auch Mischungen aus anionischen bzw. potentiell anionischen Hydrophilierungsmitteln und nichtionischen Hydrophilierungsmitteln verwendet werden.

In Weiterbildung der Erfindung ist vorgesehen, dass die aminofunktionelle Kettenverlängerer-Komponente d) $\geq$ 85 mol%, bevorzugt $\geq$ 95 mol% und besonders bevorzugt 100 mol% IPDA umfassen kann.

Als weitere Bestandteile der aminofunktionelle Kettenverlängerer d) können neben IPDI weitere $NH_2$- und / oder NH-funktionelle Verbindungen eingesetzt werden.

Bevorzugt wird die Kettenverlängerung/-terminierung vor der Dispergierung in Wasser durchgeführt, dabei reagieren die Isocyanatgruppen mit dem Kettenverlängerer zu Harnstoffgruppen.

Geeignete Komponenten - die zusätzlich zu IPDA in einem molaren Anteil von weniger als 25% eingesetzt werden können - sind Di- oder Polyamine wie 1,2-Ethylendiamin, 1,2- und 1,3-Diaminopropan, 1,4-Diaminobutan, 1,6-Diaminohexan, 2,2,4- und 2,4,4-Trimethylhexamethylendiamin, 2-Methylpentamethylendiamin, Diethylentriamin, Triaminononan, 1,3- und 1,4-Xylylendiamin, $\alpha,\alpha,\alpha',\alpha'$-Tetramethyl-1,3- und -1,4-xylylendiamin und 4,4-Diaminodicyclohexylmethan und/oder Dimethylethylendiamin. Ebenfalls möglich sind Hydrazin oder sowie Hydraziden wie Adipinsäuredihydrazid.

Zur Herstellung des erfindungsgemäßen Polyurethanharnstoffs können außer den Komponten a) bis d) auch weitere Bausteine eingesetzt werden.

Beispiele sind hydroxyfunktionellen Verbindungen mit Molekulargewichten von 62 bis 399 g/mol wie beispielsweise Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit. Weiterhin können monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin.

Bevorzugt werden außer den Komponenten a) bis d) keine weiteren Bausteine zur Herstellung des erfindungsgemäßen Polyurethanharnstoffs eingesetzt.

Die Herstellung des erfindungsgemäßen Polyurethanharnstoffs kann nach den dem Fachmann bekannten Verfahren in einer oder mehreren Stufe/-n in homogener oder bei mehrstufiger Umsetzung erfolgen und teilweise in disperser Phase durchgeführt werden. Bevorzugt erfolgt nach vollständig oder teilweise durchgeführter Polyaddition aus a) bis d) ein Dispergier-, Emulgier- oder Lösungsschritt. Im Anschluss erfolgt gegebenenfalls eine weitere Polyaddition oder Modifikation in disperser oder gelöster (homogener) Phase. Dabei können alle aus dem Stand der Technik

bekannten Verfahren wie z. B. Prepolymer-Mischverfahren, Acetonverfahren oder Schmelzdispergierverfahren verwendet werden. Bevorzugt wird nach dem Aceton-Verfahren gearbeitet.

Ein weiterer Gegenstand der vorliegenden Erfindung ist die Verwendung eines erfindungsgemäßen Polyurethanharnstoffs in der Kosmetik, bevorzugt im Bereich der Haarkosmetik, besonders bevorzugt im Bereich des Haarstylings.

Noch ein weiterer Gegenstand der vorliegenden Erfindung ist eine kosmetische Zusammensetzung, umfassend ein Lösungsmittelgemisch und einen erfindungsgemäßen Polyurethanharnstoff, wobei das Lösungsmittelgemisch Ethanol und Wasser umfasst.

Das Lösungsmittelgemisch kann gegebenenfalls weitere kosmetisch geeignete Lösungsmittel enthalten. Bevorzugte Lösungsmittel sind aliphatische Alkohole mit C2-4 Kohlenstoffatomen wie Isopropanol, t-Butanol, n-Butanol; Polyole wie Propylenglycol, Glycerin, Ethylenglycol und Polyolether; Aceton; unverzweigte oder verzweigte Kohlenwasserstoffe wie Pentan, Hexan, Isopentan und zyklische Kohlenwasserstoffe wie Cyclopentan und Cyclohexan; und deren Gemischen.

Das Lösungsmittelgemisch kann ≥ 10 Gew.-% und ≤ 98 Gew.-%, bevorzugt ≥ 15 Gew.-% und ≤ 98 Gew.-%, weiter bevorzugt ≥ 20 Gew.-% und ≤ 90 Gew.-% und besonders bevorzugt ≥ 20 Gew.-% und ≤ 80 Gew.-% Ethanol umfassen.

Ebenfalls möglich ist, dass das Lösungsmittelgemisch aus Wasser und Ethanol besteht.

Gemäß einer Ausführungsform der erfindungsgemäßen Zusammensetzung ist vorgesehen, dass sie ≥ 0,1 Gew.-% und ≤ 30 Gew.-% , bevorzugt ≥ 0,1 Gew.-% und ≤ 20 Gew.-%, weiter bevorzugt ≥ 0,5 Gew.-% und ≤ 15 Gew.-% und besonders bevorzugt ≥ 0,5 Gew.-% und ≤ 10 Gew.-% des Polyurethanharnstoffs umfasst.

Bevorzugt ist auch eine Zusammensetzung, die ≥ 10 Gew.-% und ≤ 98 Gew.-% , bevorzugt ≥ 20 Gew.-% und ≤ 98 Gew.-%, weiter bevorzugt ≥ 30 Gew.-% und ≤ 98 Gew.-% und besonders bevorzugt ≥ 40 Gew.-% und ≤ 98 Gew.-% des Lösungsmittelgemisches umfasst.

Die Trübungswerte der Systeme werden mittels eines Trübungsmessgerätes Modell 2100 P der Firma Hach (Trübungsmessung von 0,01 bis 1000 NTU) gemessen. Vor der Messung wurde das Gerät mit Formazinstandards (Standard 1 / 20 NTU, Standard 2 / 100 NTU und Standard 3 / 800 NTU) kalibriert. Gewünscht sind möglichst geringe Werte, da diese eine geringe Trübung stehen.

Ein Gegenstand der Erfindung ist auch die Verwendung einer erfindungsgemäßen kosmetischen Zusammensetzung in der Kosmetik, bevorzugt im Bereich der Haarkosmetik, besonders bevorzugt im Bereich des Haarstylings.

Noch ein weiterer Gegenstand der vorliegenden Erfindung ist ein Verfahren zum Formen von Frisuren, bei dem eine erfindungsgemäße kosmetische Zusammensetzung auf Haare aufgetragen wird.

Die erfindungsgemäße Zusammensetzung kann neben dem oben beschriebenen Polyurethan weitere geeignete Filmbildner enthalten, welche insbesondere auch zur Festigung und zum Styling der Haare beitragen können.

Der Anteil eines oder mehrerer weiterer Filmbildner kann von 0 bis 20 Gew-% und insbesondere 0 bis 10 Gew-% bezogen auf die gesamte Formulierung betragen.

Vorteilhaft werden der oder die weiteren Filmbildner aus der Gruppe der nichtionischen, anionischen, amphoteren und / oder kationischen Polymere und Mischungen hieraus ausgewählt..

Geeignete nichtionische Polymere, die in der erfindungsgemäßen Zusammensetzung alleine oder in Mischungen, vorzugsweise auch mit anionischen und / oder amphoteren und / oder zwitterionischen Polymeren enthalten sein können, sind ausgewählt aus der Gruppe:

- Polyalkyloxazoline;
- Vinylacetat Homo- oder Copolymerisate, wobei. hierzu beispielweise Copolymerisate aus Vinylacetat und Acrylsäureester, Copolymerisate aus Vinylacetat und Ethylen, Copolymerisate aus Vinylacetat und Maleinsäureester gehören;
- Acrylsäureester Copolymerisate wie z. B. die Copolymerisate aus Alkyl-Acrylat und Alkyl-Methacrylat, Copolymerisate aus Alkyl-Acrylat und Urethanen;
- Copolymerisate aus Acrylnitril und nichtionischem monomer ausgewählt aus Butadien und (Meth)Acrylat;
- Styrol Homo- und Copolymerisate, wobei hierzu beispielweise Homopolystyrol, Copolymerisate aus Styrol und Alkyl-(Meth)Acrylat , Coplymerisate aus Styrol, AlkylMethacrylat und Alkyl-Acrylat, Copolymerisate aus Styrol und Butadien, Copolymerisate aus Styrol, Butadien und Vinylpyridin gehören;
- Polyamide;

- Vinyllactame Homo- oder Copolymere, wie Vinylpyrrolidon-Homo- oder Copolymerisate; wobei hierzu beispielweise Polyvinylpyrrolidon, Polyvinylcaprolactam, Copolymerisate aus N-Vinylpyrrolidon und Vinylacetat und/oder Vinylpropionat in verschiedenen Konzentrationsverhältnissen, Polyvinylcaprolactam, Polyvinylamide und deren Salze sowie Copolymere aus Vinylpyrrolidon und Dimethylaminoethyl-methacrylat, Terpolymere aus Vinylcaprolactam, Vinylpyrrolidon und Dimethylaminoethylmethacrylat gehören;

- Polysiloxane; und

- Homopolymere des N-Vinylformamids.

Besondere bevorzugte nichtionische Polymere sind Acrylsäureester Copolymerisate, Homopolymere des Vinylpyrrolidons und Copolymere sowie Polyvinylcaprolactam.

Ganz besondere bevorzugte nichtionische Polymere sind Homopolymere des Vinylpyrrolidons z. B. Luviskol® K der Firma BASF, Copolymerisate aus Vinylpyrrolidon und Vinylacetat z. B. Luviskol® VA Typen der Firma BASF oder PVPVA® S630L der Firma ISP, Terpolymere aus Vinylpyrrolidon, Vinylacetat und Propionat wie z. B. Luviskol® VAP von BASF und Polyvinylcaprolactame z.B. Luviskol® PLUS der Firma BASF.

Vorteilhafte anionische Polymere sind Homo-oder Copolymere mit Säuregruppen enthaltenden Monomereinheiten, die gegebenenfalls mit Comonomeren, die keine Säuregruppen enthalten copolymerisiert sind. Geeignete Monomere sind ungesättigte, radikalisch polymerisierbare Verbindungen, die mindestens eine Säuregruppe besitzen, und insbesondere Carbonsäure, Sulfonsäure oder Phosphonsäure.

Beispiele für anionische Polymere, die Carbonsäuregruppen enthalten sind:

- Acrylsäure oder Methacrylsäure Homo- oder Copolymere oder deren Salze. Hierzu gehören beispielweise die Copolymerisate aus Acrylsäure und Acrylamide und / oder deren Natriumsalze, Copolymerisate aus Acrylsäure und / oder Methacrylsäure und einem ungesättigten Monomer ausgewählt aus der Gruppe Ethylene, Styrol, Vinylester, Acrylsäureester, Methacrylsäureester, gegebenenfalls ethoxylierten Verbindungen, Copolymerisate aus Vinylpyrrolidone, Acrylsäure und C1-C20 Alkyl Methacrylate z.B. Acrylidone® LM der Firma ISP, Copolymerisate aus Methacrylsäure, Ethylacrylate und Tert-butyl Acrylate z.B. Luvimer® 100 P der Firma BASF;

- Crotonsäurederivat-Homo- oder Copolymere oder deren Salze. Hierzu gehören beispielweise Vinylacetat / Crotonsäure-, Vinylacetat/Acrylat- und / oder Vinylacetat / Vinylneodecanoat / Crotonsäure-Copolymere z.B. Resyn® 28-1310 oder Resyn® 28-2930 der Firma AkzoNobel oder Luviset® CAN der Firma BASF, Natriumacrylat/Vinylalkohol-Copolymere;

- ungesättigte C4-C8 Carbonsäurederivate oder Carbonsäureanhydrid Copolymere ausgewählt aus Copolymerisaten aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus der Gruppe Vinylester, Vinylether, Vinylhalogenderivate, Phenylvinylderivate, Acrylsäure, Acrylsäureester oder Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus der Gruppe Allylester, Methallylester und ggfs. Acrylamide, Methacrylamide, alpha-Olefin, Acrylsäureester, Methacrylsäureester, Vinylpyrrolidone. Weitere bevorzugte Polymere sind Methylvinylether/Maleinsäure-Copolymere, die durch Hydrolyse von Vinylether / Maleinsäureanhydrid-Copolymeren entstehen. Diese Polymere können auch teilverestert (Ethyl, Isopropyl- bzw. Butylester) oder teilamidiert sein;

- in Wasser lösliche oder dispergierbare anionische Polyurethane, z. B. Luviset ®PUR der BASF, die von den erfindungsgemäßen Polyurethanen verschieden sind.

Vorteilhafte anionische Polymere enthaltend Sulfonsäuregruppe sind Salze von Polyvinylsulfonsäuren, Polystyrolsulfonsäuren wie z. B. Natriumpolystyrolsulfonat oder von Polyacrylamidesulfonsäuren.

Besonders vorteilhafte anionische Polymere sind Acrylsäure Copolymere, Crotonsäurederivat-Copolymer, Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus der Gruppe Vinylester, Vinylether, Vinylhalogenderivate, Phenylvinylderivate, Acrylsäure, Acrylsäureester und Salze von Polystyrolsulfonsäuren.

Ganz besondere vorteilhafte anionische Polymere sind Acrylat-Copolymere, z.B. Luvimer® der BASF, Ethylacrylat / N-tert.-Butylacrylamid / Acrylsäure-Copolymere ULTRAHOLD® STRONG der BASF, VA / Crotonat / Vinylneodecanoat-Copolymer z. B. Resyn® 28-2930 der AkzoNobel, Copolymerisate wie. Z. Copolymerisate aus Methylvinylether und Maleinsäureanhydrid teilverestert z.B. GANTREZ® der ISP und Natrium-Polystyrol-Sulfonate z. B. Flexan® 130 der AkzoNobel.

Vorteilhafte amphotere Polymere können unter den Polymeren ausgewählt werden, die statistisch in der Polymerkette verteilte Einheiten A und B enthalten, wobei A für eine Einheit steht, die von einem Monomer mit mindestens einem basischen Stickstoffatom abgeleitet ist, und B eine Einheit darstellt, die von einem sauren Monomer stammt, das eine oder mehrere Carboxy- oder Sulfonsäuregruppen aufweist. Alternativ können A und B Gruppen sein, die von zwitterionischen Carboxybetainmonomeren oder Sulfobetainmonomeren abgeleitet sind. A und B können auch eine kationische Polymerkette sein, die primäre, sekundäre, tertiäre oder quartanäre Gruppen enthalten, wobei mindestens eine Aminogruppe eine Carboxygruppe oder Sulfonsäuregruppe trägt, die über eine Kohlenwasserstoffgruppe gebunden ist, oder A und Bsind Teil einer Polymerkette mit Ethylen-$\alpha,\beta$-dicarbonsäureeinheit, bei der die Carbonsäuregruppen mit einem Polyamin umgesetzt wurden, das eine oder mehrere primäre oder sekundäre Aminogruppen enthält.

Besondere vorteilhafte amphotore Polymere sind:

- Polymere, die bei der Copolymerisation eines von einer Vinylverbindung mit Carboxygruppe abgeleiteten Monomers, wie insbesondere Acrylsäure, Methacrylsäure, Maleinsäure, $\alpha$-Chloracrylsäure, und einem basischen Monomer gebildet werden. Das basische Monomer ist von einer Vinylverbindung abgeleitet, die substituiert ist und mindestens ein basisches Atom enthält, wie insbesondere Dialkylaminoalkylmethacrylat und -acrylat, Dialkylaminoalkylmethacrylamid und -acrylamid. Solche Verbindungen sind in dem amerikanischen Patent US 3,836,537 beschrieben worden.

- Polymere mit Einheiten, die von: a) mindestens einem Monomer, das unter den Acrylamiden oder Methacrylamiden ausgewählt ist, die am Stickstoffatom mit einer Alkylgruppe substituiert sind, b) mindestens einem sauren Comonomer, das eine oder mehrere reaktive Carboxygruppen enthält, und c) mindestens einem basischen Comonomer, wie Estern von Acrylsäure und Methacrylsäure mit primären, sekundären, tertiären und quartären Aminosubstituenten und dem Quaternisierungsprodukt von Dimethylaminoethylmethacrylat mit Dimethylsulfat oder Diethylsulfat abgeleitet sind.

Besonders bevorzugte N-substituierte Acrylamide oder Methacrylamide sind Verbindungen, deren Alkylgruppen 2 bis 12 Kohlenstoffatome enthalten. Ganz besonders bevorzugt sind N-Ethylacrylamid, N-t-Butylacrylamid, N-t-Octylacrylamid, N-Octylacrylamid, N-Decylacrylamid, N-Dodecylacrylamid sowie die entsprechenden Methacrylamide.

Geeignete saure Comonomere sind insbesondere aus der Gruppe Acrylsäure, Methacrylsäure, Crotonsäure, Itaconsäure, Maleinsäure, Fumarsäure sowie den Alkylmonoestern mit 1 bis 4 Kohlenstoffatomen von Maleinsäure, Maleinsäureanhydrid, Fumarsäure oder Fumarsäureanhydrid ausgewählt.

Bevorzugte basische Comonomere sind Aminoethylmethacrylat, Butylaminoethylmethacrylat, N,N-Dimethylaminoethylmethacrylat, N-t-Butylaminoethylmethacrylat.

- Vernetzte und ganz oder teilweise acylierte Polyaminoamide, die von Polyaminoamiden der folgenden allgemeinen Formel

$$-[CO-R-CO-Z]-$$

abgeleitet sind, worin R eine zweiwertige Gruppe ist, die von einer gesättigten Dicarbonsäure, einer aliphatischen Mono- oder Dicarbonsäure mit ethylenischer Doppelbindung, einem Ester dieser Säuren mit einem niederen Alkanol mit 1 bis 6 Kohlenstoffatomen oder einer Gruppe abgeleitet ist, die bei der Addition einer dieser Säuren an ein bisprimäres oder bis-sekundäres Amin entsteht, und Z eine Gruppe ist, die von einem bis-primären, mono- oder bis-sekundären Polyalkylenpolyamin abgeleitet ist

Die gesättigten Carbonsäuren sind vorzugsweise unter den Säuren mit 6 bis 10 Kohlenstoffatomen, wie Adipinsäure, 2,2,4-Trimethyladipinsäure und 2,4,4,-Trimethyladipinsäure, Terephthalsäure; Säuren mit ethylenischer Doppelbindung, wie beispielsweise Acrylsäure, Methacrylsäure und Itaconsäure ausgewählt.

Die bei der Acylierung verwendeten Alkansultone sind vorzugsweise Propansulton oder Butansulton, die Salze der Acylierungsmittel sind vorzugsweise die Natriumsalze oder Kaliumsalze.

- Polymeren mit zwitterionischen Einheiten der folgenden Formel:

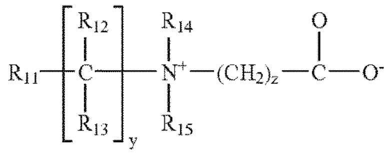

worin $R_{11}$ eine polymerisierbare ungesättigte Gruppe, wie Acrylat, Methacrylat, Acrylamid oder Methacrylamid, y und z ganze Zahlen von 1 bis 3, $R_{12}$ und $R_{13}$ ein Wasserstoffatom, Methyl, Ethyl oder Propyl, $R_{14}$ und $R_{15}$ ein Wasserstoffatom oder eine Alkylgruppe bedeuten, die so gewählt ist, dass die Summe der Kohlenstoffatome $R_{14}$ und $R_{15}$ 10 nicht übersteigt, sind.
Polymere, die solche Einheiten enthalten, können auch Einheiten aufweisen, die von nicht zwitterionischen Monomeren stammen, wie Dimethyl- und Diethylaminoethylacrylat oder Dimethyl- und Diethylaminoethylmethacrylat oder Alkylacrylate oder Alkylmethacrylate, Acrylamide oder Methacrylamide oder Vinylacetat.

- Polymere, die von Chitosan abgeleitet sind und Monomereinheiten enthalten, die den folgenden Formeln entsprechen:

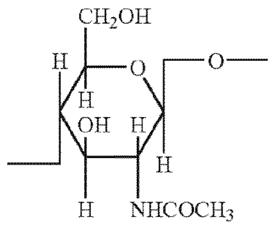

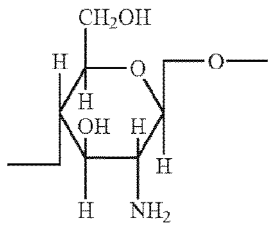

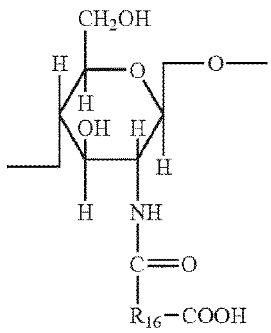

wobei die erste Einheit in Mengenanteilen von 0 bis 30 %, die zweite Einheit in Mengenanteilen von 5 bis 50 % und die dritte Einheit in Mengenanteilen von 30 bis 90 % enthalten ist, mit der Maßgabe, dass in der dritten Einheit $R_{16}$ eine Gruppe der folgenden Formel bedeutet:

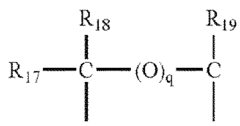

worin falls q = 0, die Gruppen $R_{17}$, $R_{18}$ und $R_{19}$, gleich oder verschieden jeweils ein Wasserstoffatom, Methyl, Hydroxy, Acetoxy oder Amino, einen Monoalkylaminrest oder einen Dialkylaminrest, die gegebenenfalls durch ein oder mehrere Stickstoffatome unterbrochen und / oder gegebenenfalls mit einer oder mehreren Gruppen Amino, Hydroxy, Carboxy, Alkylthio, Sulfonsäure, Alkylthio, dessen Alkylgruppe einen Aminorest trägt, wobei mindestens eine der Gruppen $R_{17}$, $R_{18}$ und $R_{19}$ in diesem Fall ein Wasserstoffatom bedeutet; oder falls q = 1, die Gruppen $R_{17}$, $R_{18}$ und $R_{19}$ jeweils ein Wasserstoffatom, sowie die Salze, die diese Verbindungen mit Basen oder Säuren bilden, sind.

- Polymere, die der folgenden allgemeinen Formel entsprechen und die beispielsweise in dem französischen Patent 1 400 366 beschrieben sind:

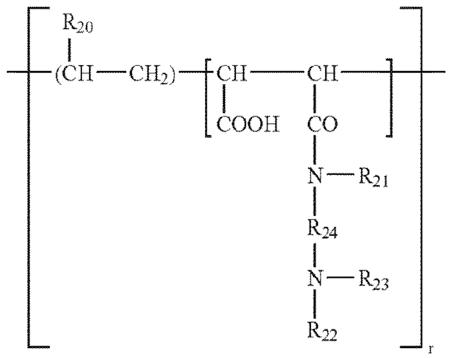

worin $R_{20}$ ein Wasserstoffatom, $CH_3O$, $CH_3CH_2O$ oder Phenyl ist, $R_{21}$ ein Wasserstoffatom oder eine niedere Alkylgruppe, wie Methyl oder Ethyl ist, $R_{22}$ ein Wasserstoffatom oder eine niedere $C_{1-6}$-Alkylgruppe, wie Methyl oder Ethyl ist, $R_{23}$ eine niedere $C_{1-6}$-Alkylgruppe, wie Methyl oder Ethyl oder eine Gruppe der Formel: $-R_{24}-N(R_{22})_2$ ist, wobei $R_{24}$ eine Gruppe $-CH_2-CH_2$, $-CH_2-CH_2-CH_2-$ oder $-CH_2-CH(CH_3)-$ darstellt und wobei $R_{22}$ die oben angegebenen Bedeutungen aufweist.

- Polymere, die bei der N-Carboxyalkylierung von Chitosan gebildet werden können, wie N-Carboxymethylchitosan oder N-Carboxybutylchitosan.

- Alkyl($C_{1-5}$)vinylether/Maleinsäureanhydrid-Copolymere, die teilweise durch Semiamidierung mit einem N,N-Dialkylaminoalkylamin wie N,N-Dimethylaminopropylamin oder einem N,N-Dialkylaminoalkohol teilweise modifiziert sind. Diese Polymere können auch weitere Comonomere enthalten, wie Vinylcaprolactam.

Ganz besondere vorteilhafte amphotere Polymere sind z.B. die Copolymere Octylacrylamid / Acrylate / Butylamino-Ethylmethacrylat-Copolymer, die unter den Bezeichnungen AMPHOMER®, AMPHOMER® LV 71 oder BALANCE® 47 der Firma AkzoNobel im Handel sind, und Methylmethacrylat / Methyl-dimethylcarboxymethylammoniumethylmethacrylat-Copolymere.

Es ist gegebenenfalls vorteilhaft, die anionischen und amphoteren Polymere zur Verbesserung ihrer Wasserlöslichkeit bzw. ihrer Wasserdispergierbarkeit mit geeigneten Basen zu neutralisieren.

Als Neutralisationsmittel für Polymere, die Säuregruppen enthalten, können folgende Basen eingesetzt werden: Hydroxide, deren Kation Ammonium oder ein Alkalimetall ist wie z. B. NaOH oder KOH.

Andere Neutralisationsmittel sind primäre, sekundäre oder tertiäre Amine, Aminoalkohole oder Ammoniak. Bevorzugt werden hier 2-Amino-2-methyl-1,3-propandiol (AMPD), 2-Amino-2-ethyl-1,3-propandiol (AEPD), 2-Amino-2-methyl-1-propanol (AMP), 2-Amino-1-butanol (AB), 2-Amino-1,3-propandiol, Monoethanolamin (MEA), Diethanolamin (DEA), Triethanolamin (TEA), Monoisopropanolamin (MIPA), Diisopropanolamin (DIPA), Triisopropanolamin (TIPA), Dimethyl Laurylamin (DML), Dimethyl Myristalamin (DMM), und Dimethyl Stearamin (DMS) verwendet.

Die Neutralisation kann je nach Anwendungszweck teilweise oder vollständig erfolgen.

Gegebenfalls einsetzbar aber jedoch weniger bevorzugt sind kationische Polymere, wie beispielweise Polymere, die primäre, sekundäre tertiäre und / oder quaternäre Aminogruppen enthalten, die Teil der Polymerkette oder direkt an die Polymerkette gebunden sind.

Die erfindungsgemäßen Zusammensetzungen können des weiteren Verdicker enthalten. Vorteilhafte Verdicker sind:

- Vernetzte oder nichtvernetzte Acrylsäure oder Methacrylsäure Homo- oder Copolymere. Hierzu gehören vernetzte Homopolymere von Methacrylsäure oder Acrylsäure, Copolymerisate aus Acrylsäure und / oder Methacrylsäure und Monomeren, die von anderen Acryl- oder Vinylmonomeren abgeleitet sind, wie C10-30 Alkylacrylate, C10-30-Alkylmethacrylate und Vinylacetat.

- Verdickende Polymere natürlicher Herkunft beispielweise auf Cellulosebasis, Guargummi, Xanthan, Scleroglucan, Gellangummi, Rhamsan und Karayagummi, Alginate, Maltodextrin, Stärke und ihre Derivate, Johannisbrotkernmehl, Hyaluronsäure.

- Nichtionische, anionische, kationische oder amphotere assoziative Polymere z.B. auf Basis von Polyethylenglycole und ihre Derivate, oder Polyurethane.

- Vernetzte oder nichtvernetzte Homopolymere oder Copolymere auf Basis von Acrylamid oder Methacrylamid, wie Homopolymere von 2-Acrylamido-2-methylpropansulfonsäure, Copolymere von Acrylamid oder Methacrylamid und Methacryloyloxyethyltrimethylammoniumchlorid oder Copolymere von Acrylamid und 2-Acrylamido-2-methylpropansulfonsäure angegeben werden.

Besondere vorteilhafte Verdicker sind verdickende Polymere natürlicher Herkunft, vernetzte Acrylsäure oder Methacrylsäure Homo- oder Copolymere und vernetzte Copolymere von 2-Acrylamido-2-methylpropansulfonsäure.

Ganz besondere vorteilhafte Verdicker sind Xanthangummi, wie die unter den Bezeichnungen Keltrol® und Kelza® von der Firma CP Kelco angebotenen Produkte oder die Produkte der Firma RHODIA mit der Bezeichnung Rhodopol und Guargummi, wie die unter der Bezeichnung Jaguar® HP105 von der Firma RHODIA erhältlichen Produkte.

Ganz besondere vorteilhafte Verdicker sind vernetzte Homopolymere von Methacrylsäure oder Acrylsäure, die von der Firma Lubrizol unter den Bezeichnungen Carbopol® 940, Carbopol® 941, Carbopol® 980, Carbopol® 981, Carbopol® ETD 2001, Carbopol® EDT 2050, Carbopol® 2984, Carbopol® 5984 und Carbopol® Ultrez 10, von der Firma 3V unter den Bezeichnungen Synthalen® K, Synthalen® L und Synthalen® MS und von der Firma PROTEX unter den Bezeichnungen Modarez® V 1250 PX, Modarez® V2000 PX, Viscaron® A1600 PE und Viscaron® A700 PE im Handel erhältlich sind.

Ganz besondere vorteilhafte Verdicker sind vernetzte Copolymere von Acrylsäure oder Methacrylsäure und einem $C_{10-30}$-Alkylacrylat oder $C_{10-30}$-Alkylmethacrylat und Copolymere von Acrylsäure oder Methacrylasäure und Vinylpyrrolidon. Solche Copolymere sind beispielsweise von der Firma Lubrizol unter den Bezeichnungen Carbopol® 1342, Carbopol® 1382, Pemulen® TR1 oder Pemulen® TR2 und von der Firma ISP unter den Bezeichnungen Ultrathix P-100 (INCI: Acrylic Acid/VP Crosspolymer) im Handel erhältlich.

Ganz besondere vorteilhafte Verdicker sind vernetzte Copolymere von 2-Acrylamido-2-methylpropansulfonsäure. Solche Copolymere sind beispielsweise von der Firma Clariant unter den Bezeichnungen Aristoflex® AVC (INCI: Ammonium Acryloyldimethyltaurat/VP Copolymer) erhältlich

Falls die Verdicker verwendet werden, sind sie im Allgemeinen in einer Konzentration von 0 % bis 2 Gew.-% vorzugsweise von 0 % bis 1 Gew.-% vorhanden.

Die erfindungsgemäßen Zusammensetzungen können des weiteren ein Treibgas enthalten.

Bevorzugte Treibgase sind Kohlenwasserstoffe wie Propan, Isobutan und n-Butan sowie deren Mischungen. Druckluft, Kohlendioxid, Stickstoff, Stickstoffdioxid und Dimethylether sowie Mischungen all dieser Gase können ebenfalls verwendet werden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte. Dabei handelt es sich, insbesondere Fluorkohlenwasserstoffe und Fluorchlorkohlenwasserstoffe (FCKW) wie z.B. 1,2-difluoroethan (Treibmittel 152 A).

In den erfindungsgemäßen Zusammensetzungen können des weiteren haarpflegende Wirkstoffe enthalten sein. Als Pflegestoffe können bevorzugt cyclische Polydimethylsiloxane (Cyclomethicone) oder Silikontenside (Polyethermodifizierte Siloxane) vom Typ Dimethicone Copolyol oder Simethicon zum Einsatz kommen. Cyclomethicone werden u. a. unter der Handelsbezeichnungen Abil® K4 von der Firma Goldschmidt oder z.B. DC 244, DC 245 oder DC 345 von der Firma Dow Corning angeboten. Dimethicon-Copolyole werden z. B. unter der Handelsbezeichnung DC 193 von der Firma Dow Corning bzw. Belsil® DM 6031 von der Firma Wacker angeboten.

Optional können herkömmliche Additive ebenfalls in der Zusammensetzung enthalten sein, beispielsweise um ihr bestimmte Modifizierungseigenschaften zu verleihen. Hierbei kann es sich etwa um Silikone oder Silikonderivate, Benetzungsmittel, Feuchthaltemittel, Weichmacher wie Glycerin, Glykol und Phthalester und -ether, Riechstoffe und Parfüms, UV-Absorber, Farbstoffe, Pigmente, und andere Farbmittel, antikorrosive Mittel, Neutralisationsmittel, Antioxidantien, Antiklebemittel, Kombinierungsmittel und Konditioniermittel, antistatische Mittel, Glanzmittel, Konservierungsmittel, Proteine und Derivate davon, Aminosäuren, Vitamine, Emulgatoren, oberflächenaktive Mittel, Viskositätsmodifizierer, Verdicker und Rheologiemodifizierer, Geliermittel, Trübungsmittel, Stabilisatoren, Tenside, Sequestierungsmittel, Komplexbildner, Perlglanzmittel, ästhetische Verstärker, Fettsäuren, Fettalkohole, Triglyceride, botanische Extrakte, Klärhilfsmittel und Filmbildner handeln.

Diese Additive sind im Allgemeinen in einer Konzentration von etwa 0,001 % bis 15 Gew.-% vorzugsweise 0,01 % bis 10 Gew.-% bezogen auf das Gesamtgewicht der Zusammensetzung vorhanden.

Die erfindungsgemäßen Zusammensetzungen können vorteilhafter Weise in einer Pumpspray- oder Aerosolverpackung vorliegen. Sie können auch vorteilhaft mit einem Treibgas aufgeschäumt werden. Dementsprechend sind Pumpsprays Aerosolverpacken und Schaumspender, die die erfindungsgemäße Zusammensetzung enthalten, ebenfalls

Bestandteil der Erfindung.

Eine weitere bevorzugte Ausführungsform der erfindungsgemäßen Zusammensetzung liegt in der Form eines Sprays vor, welches zusätzlich einen oder mehrere der folgenden Bestandteile enthält: kosmetisch geeignete Lösungsmittel, wie aliphatische Alkohole mit 2-4 Kohlenstoffatomen, bevorzugt Ethanol, Polyole, Aceton, unverzweigte oder verzweigte Kohlenwasserstoffe, zyklische Kohlenwasserstoffe und deren Gemische, sowie Treibgase wie Kohlenwasserstoffe, Druckluft, Kohlendioxid, Stickstoff, Stickstoffdioxid, Dimethylether, Fluorkohlenwasserstoffe und Fluorchlorkohlenwasserstoffe, bevorzugt Dimethylether und/oder ein Propan/Butan-Gemisch.

Die vorliegende Erfindung wird an Hand der folgenden Beispiele erläutert. Alle Mengenangaben, Anteile und Prozentanteile sind, soweit nicht anders angegeben, auf das Gewicht und die Gesamtmenge bzw. auf das Gesamtgewicht der Zusammensetzung bezogen.

## Beispiele:

Sofern nicht abweichend gekennzeichnet, beziehen sich alle Prozentangaben auf das Gewicht.

Sofern nicht abweichend vermerkt, beziehen sich alle analytischen Messungen auf Messungen bei Temperaturen von 23 °C.

Die Feststoff- bzw. Festkörpergehalte wurden entsprechend DIN EN ISO 3251 durch Erhitzen einer ausgewogenen Probe auf 105 °C bis zur Gewichtskonstanz ermittelt. Bei Gewichtskonstanz wurde durch erneutes Auswiegen der Probe der Festkörpergehalt berechnet.

NCO-Werte wurden, wenn nicht ausdrücklich anders erwähnt, volumetrisch gemäß DIN-EN ISO 11909 bestimmt.

Die Kontrolle auf freie NCO-Gruppen wurde mittels IR-Spektroskopie (Bande bei 2260 $cm^{-1}$) durchgeführt.

Die angegebenen Viskositäten wurden mittels Rotationsviskosimetrie nach DIN 53019 bei 23 °C mit einem Rotationsviskosimeter der Firma Anton Paar Germany GmbH, Ostfildern, DE bestimmt.

Die Bestimmung der mittleren Teilchengrößen (angegeben ist das Zahlenmittel) der Polyurethan-Dispersionen erfolgte nach Verdünnen mit entionisiertem Wasser mittels Laserkorrelations-Spektroskopie (Gerät: Malvern Zetasizer 1000, Malver Inst. Limited).

### Verwendete Substanzen und Abkürzungen:

| | |
|---|---|
| Diaminosulfonat: | $NH_2$-$CH_2CH_2$-NH-$CH_2CH_2$-$SO_3Na$ (45 %ig in Wasser) |

### Erfindungsgemäßes Beispiel: Polyurethanharnstoff-Dispersion A

1360,0 g eines Polyesters aus Adipinsäure, Hexandiol und Neopentylglykol mit einem mittleren Molekulargewicht von 1700 g/mol wurden auf 65 °C aufgeheizt. Anschließend wurden 318,5 g Isophorondiisocyanat (IPDI) zugegeben und solange bei 105 °C gerührt bis der theoretische NCO-Wert unterschritten war. Das fertige Prepolymer wurde mit 3000 g Aceton bei 50 °C gelöst und anschließend eine Lösung aus 23,4 g Isophorondiamin (IPDA), 129,6 g Diaminosulfonat und 357 g Wasser zudosiert. Die Nachrührzeit betrug 15 min. Danach wurde durch Zugabe von 2900 g Wasser dispergiert. Es folgte die Entfernung des Lösemittels durch Destillation im Vakuum und es wurde eine lagerstabile Dispersion erhalten, der Festgehalt wurde durch Zusatz von Wasser eingestellt.

| | |
|---|---|
| Feststoffgehalt: | 32 % |
| Partikelgröße (LKS): | 27 nm |
| Viskosität: | 1500 mPas |
| pH-Wert: | 7,3 |

### Erfindungsgemäßes Beispiel: Polyurethanharnstoff-Dispersion B

318,8 g eines Polyesters aus Adipinsäure, Hexandiol und Neopentylglykol mit einem mittleren Molekulargewicht von 1700 g/mol wurden auf 65 °C aufgeheizt. Anschließend wurden 70,9 g Isophorondiisocyanat zugegeben und solange bei 105 °C gerührt bis der theoretische NCO-Wert unterschritten war. Das fertige Prepolymer wurde mit 700 g Aceton bei 50°C gelöst und anschließend eine Lösung aus 3,5 g Isophorondiamin, 30,4 g Diaminosulfonat und 84 g Wasser zudosiert. Die Nachrührzeit betrug 15 min. Danach wurde durch Zugabe von 513 g Wasser dispergiert. Es folgte die Entfernung des Lösemittels durch Destillation im Vakuum und es wurde eine lagerstabile Dispersion erhalten, der Festgehalt wurde durch Zusatz von Wasser eingestellt.

| | |
|---|---|
| Feststoffgehalt: | 30 % |
| Partikelgröße (LKS): | 32 nm |
| Viskosität: | 1000 mPas |
| pH-Wert: | 7,2 |

**Erfindungsgemäßes Beispiel: Polyurethanharnstoff-Dispersion C**

319 g eines Polyesters aus Adipinsäure, Hexandiol und Neopentylglykol mit einem mittleren Molekulargewicht von 1700 g/mol wurden auf 65 °C aufgeheizt. Anschließend wurden 79,2 g Isophorondiisocyanat zugegeben und solange bei 105 °C gerührt bis der theoretische NCO-Wert unterschritten war. Das fertige Prepolymer wurde mit 1100 g Aceton bei 50°C gelöst und anschließend eine Lösung aus 8,0 g Isophorondiamin, 30,4 g Diaminosulfonat und 84 g Wasser zudosiert. Die Nachrührzeit betrug 15 min. Danach wurde durch Zugabe von 540 g Wasser dispergiert. Es folgte die Entfernung des Lösemittels durch Destillation im Vakuum und es wurde eine lagerstabile Dispersion erhalten, der Festgehalt wurde durch Zusatz von Wasser eingestellt.

| | |
|---|---|
| Feststoffgehalt: | 28 % |
| Partikelgröße (LKS): | 35 nm |
| Viskosität: | 760 mPas |
| pH-Wert: | 7,7 |

**Vergleichsbeispiel: Polyurethanharnstoff-Dispersion D**

319 g eines Polyesters aus Adipinsäure, Hexandiol und Neopentylglykol mit einem mittleren Molekulargewicht von 1700 g/mol wurden auf 65 °C aufgeheizt. Anschließend wurden 56,0 g Isophorondiisocyanat und 14,1 g Hexamethylendiisocyanat zugegeben und solange bei 105°C gerührt bis der theoretische NCO-Wert unterschritten war. Das fertige Prepolymer wurde mit 690 g Aceton bei 50 °C gelöst und anschließend eine Lösung aus 5,5 g Isophorondiamin, 30,4 g Diaminosulfonat und 84 g Wasser zudosiert. Die Nachrührzeit betrug 15 min. Danach wurde durch Zugabe von 520 g Wasser dispergiert. Es folgte die Entfernung des Lösemittels durch Destillation im Vakuum und es wurde eine lagerstabile Dispersion erhalten, der Festgehalt wurde durch Zusatz von Wasser eingestellt.

| | |
|---|---|
| Feststoffgehalt: | 31 % |
| Partikelgröße (LKS): | 71 nm |
| Viskosität: | 210 mPas |
| pH-Wert: | 7,4 |

**Vergleichsbeispiel: Polyurethanharnstoff-Dispersion E**

319 g eines Polyesters aus Adipinsäure, Hexandiol und Neopentylglykol mit einem mittleren Molekulargewicht von 1700 g/mol wurden auf 65 °C aufgeheizt. Anschließend wurden 74,6 g Isophorondiisocyanat zugegeben und solange bei 105 °C gerührt bis der theoretische NCO-Wert unterschritten war. Das fertige Prepolymer wurde mit 700 g Aceton bei 50°C gelöst und anschließend eine Lösung aus 4,1 g Isophorondiamin, 30,4 g Diaminosulfonat, 0,5 g Ethylendiamin und 87 g Wasser zudosiert. Die Nachrührzeit betrug 15 min. Danach wurde durch Zugabe von 520 g Wasser dispergiert. Es folgte die Entfernung des Lösemittels durch Destillation im Vakuum und es wurde eine lagerstabile Dispersion erhalten, der Festgehalt wurde durch Zusatz von Wasser eingestellt.

| | |
|---|---|
| Feststoffgehalt: | 30 % |
| Partikelgröße (LKS): | 47 nm |
| Viskosität: | 115 mPas |
| pH-Wert: | 7,6 |

**Vergleichsbeispiel: Polyurethanharnstoff-Dispersion F**

319 g eines Polyesters aus Adipinsäure, Hexandiol und Neopentylglykol mit einem mittleren Molekulargewicht

von 1700 g/mol wurden auf 65 °C aufgeheizt. Anschließend wurden 74,6 g Isophorondiisocyanat zugegeben und solange bei 105 °C gerührt bis der theoretische NCO-Wert unterschritten war. Das fertige Prepolymer wurde mit 700 g Aceton bei 50°C gelöst und anschließend eine Lösung aus 3,7 g Isophorondiamin, 30,4 g Diaminosulfonat, 2,2 g Methylen-bis(4-aminocyclohexan) und 100 g Wasser zudosiert. Die Nachrührzeit betrug 15 min. Danach wurde durch Zugabe von 510 g Wasser dispergiert. Es folgte die Entfernung des Lösemittels durch Destillation im Vakuum und es wurde eine lagerstabile Dispersion erhalten, der Festgehalt wurde durch Zusatz von Wasser eingestellt.

| | |
|---|---|
| Feststoffgehalt: | 30 % |
| Partikelgröße (LKS): | 38 nm |
| Viskosität: | 70 mPas |
| pH-Wert: | 6,7 |

**Vergleichsbeispiel: Polyurethanharnstoff-Dispersion G**

319 g eines Polyesters aus Adipinsäure, Hexandiol und Neopentylglykol mit einem mittleren Molekulargewicht von 1700 g/mol wurden auf 65 °C aufgeheizt. Anschließend wurden 74,6 g Isophorondiisocyanat zugegeben und solange bei 105 °C gerührt bis der theoretische NCO-Wert unterschritten war. Das fertige Prepolymer wurde mit 700 g Aceton bei 50°C gelöst und anschließend eine Lösung aus 30,4 g Diaminosulfonat, 6,8 g Methylen-bis(4-aminocyclohexan) und 100 g Wasser zudosiert. Die Nachrührzeit betrug 15 min. Danach wurde durch Zugabe von 510 g Wasser dispergiert. Es folgte die Entfernung des Lösemittels durch Destillation im Vakuum und es wurde eine lagerstabile Dispersion erhalten, der Festgehalt wurde durch Zusatz von Wasser eingestellt.

| | |
|---|---|
| Feststoffgehalt: | 31 % |
| Partikelgröße (LKS): | 42 nm |
| Viskosität: | 70 mPas |
| pH-Wert: | 6,7 |

**Vergleichsbeispiel: Polyurethanharnstoff-Dispersion H**

319 g eines Polyesters aus Adipinsäure, Hexandiol und Neopentylglykol mit einem mittleren Molekulargewicht von 1700 g/mol wurden auf 65 °C aufgeheizt. Anschließend wurden 88,1 g Methylen-bis (4-cyclohexylisocyanat) zugegeben und solange bei 105 °C gerührt bis der theoretische NCO-Wert unterschritten war. Das fertige Prepolymer wurde mit 730 g Aceton bei 50°C gelöst und anschließend eine Lösung aus 5,5 g Isophorondiamin, 30,4 g Diaminosulfonat und 84 g Wasser zudosiert. Die Nachrührzeit betrug 15 min. Danach wurde durch Zugabe von 540 g Wasser dispergiert. Es folgte die Entfernung des Lösemittels durch Destillation im Vakuum und es wurde eine lagerstabile Dispersion erhalten, der Festgehalt wurde durch Zusatz von Wasser eingestellt.

| | |
|---|---|
| Feststoffgehalt: | 40 % |
| Partikelgröße (LKS): | 300 nm |
| Viskosität: | $<$ 50 mPas |
| pH-Wert: | 8,8 |

**Vergleichsbeispiel: Polyurethanharnstoff-Dispersion I**

340 g eines Polyesters aus Adipinsäure, Hexandiol und Neopentylglykol mit einem mittleren Molekulargewicht von 1700 g/mol wurden auf 65 °C aufgeheizt. Anschließend wurden 60,1 g Hexamethylendiisocyanat zugegeben und solange bei 105°C gerührt bis der theoretische NCO-Wert unterschritten war. Das fertige Prepolymer wurde mit 711 g Aceton bei 50 °C gelöst und anschließend eine Lösung aus 2,1 g Ethylendiamin, 32,4 g Diaminosulfonat und 104,3 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten, der Festgehalt wurde durch Zusatz von Wasser eingestellt.

| | |
|---|---|
| Feststoffgehalt: | 40 % |
| Partikelgröße (LKS): | 198 nm |
| Viskosität: | 700 mPas |

(fortgesetzt)

| | |
|---|---|
| pH-Wert: | 6,31 |

**Anwendungstechnische Vergleichversuche:**

Trübungsmessung:

Die optischen Eigenschaften der erfindungsgemäßen Polyurethanharnstoffe wurden in drei unterschiedlichen Mischungen untersucht:

| | |
|---|---|
| a) VOC 80 Mischungen: | 2 Gew. % des jeweiligen Polyurethanharnstoffs (bezogen auf Feststoff) + 80 % Ethanol (vergällt mit 0,448 % Diethylphtalate) + Wasser zu 100 % ergänzt |
| b) VOC 55 Mischungen : | 2 Gew. % des jeweiligen Polyurethanharnstoffs (bezogen auf Feststoff) + 55 % Ethanol (vergällt mit 0,448 % Diethylphtalate) + Wasser zu 100 % ergänzt |
| c) VOC 0 Mischungen: | 2 Gew. % des jeweiligen Polyurethanharnstoffs (bezogen auf Feststoff) + Wasser zu 100 % ergänzt |

Die Trübung der jeweiligen Mischungen wurde 1 Tag nach der Herstellung mittels eines Trübungsmessgerätes Modell 2100 P der Firma Hach (Trübungsmessung von 0,01 bis 1000 NTU) gemessen. Vor der Messung wurde das Gerät mit Formazinstandards (Standard 1 / 20 NTU, Standard 2 / 100 NTU und Standard 3 / 800 NTU) kalibriert.

Die Ergebnisse der Trübungsmessungen sind in der nachfolgenden Tabelle 1 zusammen gefasst:

Tabelle 1

| Polyurethanharnstoff | VOC 80 | VOC 55 | VOC 0 |
|---|---|---|---|
| | Trübungswerte in_NTU | | |
| A | 13 | 39 | 82 |
| B | 74 | 40 | 109 |
| C | 43 | 46 | 119 |

Die Ergebnisse der Trübungsmessungen von Mischungen der Polyurethanharnstoffe der Vergleichsbeispiele sind in der Tabelle 2 wiedergegeben.

| Polyurethanharnstoff | VOC 80 | VOC 55 | VOC 0 |
|---|---|---|---|
| | Trübungswerte in NTU | | |
| D | 80 | 102 | 282 |
| E | 134 | 60 | 147 |
| F | 105 | 65 | 115 |
| G | 121 | 63 | 173 |
| H | n.m (> 1000) | n.m (> 1000) | n.m (> 1000) |

Die Versuche zeigen deutlich, dass aus den erfindungsgemäßen Polyurethanharnstoffen Mischungen mit besonders niedrigen Trübungswerte erhältlich sind; d.h. sie eigenen sich besonders gut zur Herstellung transparenter Mischungen.

**Curl Retention:**

Für die sogenannten "Curl Retention"-Versuche wurden kommerziell erhältliche europäische Mischhaare (Nutzlänge: 16 cm, Kerling, Gewicht: 0,7 g) verwendet. Die Haare wurden vor der Anwendung einer standardisierten Waschprozedur unterzogen. Dazu wurde die 15 Minuten in Wasser eingeweichten Haare mit 0,2 mL Standardshampoo eine Minute shampooniert, gründlich mit warmen Wasser ausgespült, kalt trocken geföhnt und bei 21 ± 1 °C und 50 ± 5 %

relativer Feuchtigkeit konditioniert. 0,1 g von einer 2 Gew % Polymermischung wurde auf die Strähne appliziert. Von den so vorbereiteten Haaren wurden Strähnen auf 16 mm Wickler gewickelt und dann bei 21 ± 1 °C und 50 ± 5 % relativer Feuchtigkeit für mindestens 18 Stunden konditioniert

Die "Curl Retention"-Versuche wurden in einer speziellen klimatisierten Kammer bei einer relativen Feuchtigkeit von 90 ± 5 % durchgeführt. Die Temperatur in der Kammer betrug 21 ± 1°C. Die vorbereiteten Strähnen wurden gleichzeitig in die Kammer gehängt. Die Länge der Strähnen wurde zu unterschiedlichen Zeiten an einer Skala abgelesen. Jeder Versuch wurde mit acht Strähnen durchgeführt.

Die "Curl Retention" wurde nach der folgenden Formel errechnet:

$$CR = (l_f - l_{ti})/(l_f - l_t) x 100 ;$$

in der $l_f$ die Strähnelänge, $l_{ti}$ ursprüngliche Länge der gelockten Haarsträhne und $l_t$ Länge der gelockten Haarsträhne zur Zeit t- waren.

Die Ergebnisse der Messung sind in der Figur 1 grafisch dargestellt. Hieraus geht deutlich hervor, dass mit dem erfindungsgemäß verwendeten Polyurethanharnstoff ein guter Lockenhalt erzielt wird.

**Anwendungstechnische Beispiele:**

Im Folgenden sind Beispiele für Kosmetische Formulierungen aufgeführt, in denen die erfindungsgemäßen Zusammensetzungen verwendet werden. Es ist jeweils angegeben, wie viel Gewichtsteile der einzelnen Komponenten in einer Formulierung vorhanden sind.

"Pump-setting-Spray"

| | A | B |
|---|---|---|
| Erfindungsgemäßer Polyurethanhamstoff (bezogen auf Feststoff) | 2 | 10 |
| Ethanol | 90 | 55 |
| Parfüm | q.s. | q.s. |
| Wasser | bis 100% | bis 100% |
| q.s. quantum satis | | |

Aerosol-Hair-Sprays

| | C | D | E | F | G |
|---|---|---|---|---|---|
| Erfindungsgemäßer Polyurethanharnstoff (bezogen auf Feststoff) | 5 | 10 | 2 | 5 | 8 |
| Octylacrylamid/acrylat/Butylaminoethyl methacrylat[1] (bezogen auf Feststoff) | | | | 1,8 | |
| Acrylat-Copolymer[2] | | | 2 | | |
| Aminomethylpropanol | | | q.s | q.s | |
| Glycerin | | 0,5 | | | |
| Panthenol | | | 0,5 | | 0,5 |
| PEG/PPG-18/18 Dimethicone | | | | 0,5 | |
| PEG-12 dimethicone | | 0,05 | | | |
| Propylene Glycol | | | | 0,5 | |
| Cyclomethicone | | | 1,0 | | 1,0 |
| Benzophenone-3 | | 0,1 | 0,1 | | 0,1 |
| Parfüm | q.s | q.s | q.s | q.s | q.s |

(fortgesetzt)

| | C | D | E | F | G |
|---|---|---|---|---|---|
| Ethanol | 30 | 25 | 70 | 50 | 50 |
| Wasser | bis 100% | bis 100% | bis 100% | bis 100% | bis 100% |
| Propan/butan 3,5 Bar. (20°C) | | | 20 | 10 | |
| Dimethylether | 40 | 30 | | 30 | |
| Fluorkohlenwasserstoff 152 A | | | | | 20 |
| [1] Amphomer, AkzoNobel<br>[2] Luvimer P-100, BASF | | | | | |

Haarschaum

| | H | I |
|---|---|---|
| Erfindungsgemäßer Polyurethanharnstoff (bezogen auf Feststoff) | 2 | 4 |
| Cilycerin | 0,1 | |
| Panthenol | 0,05 | 0,5 |
| Polyquaternium-164 | 2 | |
| Cetyltrimethylammoniumchlorid | 0,2 | 0,5 |
| PEG-12 dimethicone | | 0,5 |
| Cyclomethicone | | 0,5 |
| Benzophenone-3 | 0,1 | |
| Parfüm | q.s. | q.s. |
| Ethanol | 5 | 10 |
| Wasser | bis 100% | bis 100% |
| Konservierungsmittel | q.s. | q.s. |
| Propan/butanDimethylether | 10 | 7 |
| Dimethylether | | 7 |
| Fluorkohlenwasserstoff 152 A | | 3 |

Haargel/creme

| | J | K | L |
|---|---|---|---|
| Erfindungsgemäßes Polyurethanharnstoff (bezogen auf Feststoff) | 5 | 2 | 8 |
| Carbomer | 0,8 | | |
| Acrylic Acidsäure/VP Crossopolymer | | 0,5 | |
| Ammonium Aacryloyldimethyltaurat/VP Copolymer | | | 0,8 |
| Glycerin | 0,5 | | |
| Panthenol | | 0,5 | 0,5 |
| Propylenglycol | | | 0,2 |
| Cyclomethicone | | 0,2 | |
| Neutralisierungsmittel | q.s. | q.s. | q.s. |

(fortgesetzt)

| | J | K | L |
|---|---|---|---|
| Parfüm | q.s. | q.s. | q.s. |
| Ethanol | 6 | | |
| Wasser | bis 100% | bis 100% | bis 100% |
| Konservierungsmittel | q.s. | q.s. | q.s. |

**Patentansprüche**

1. Polyurethanharnstoff erhältlich durch Umsetzung

   a) einer einzigen Polyisocyanat-Komponente,
   b) wenigstens einer polymeren Polyolkomponente,
   c) wenigstens einer hydrophilierenden Komponente und
   d) einer einzigen aminofunktionellen Kettenverlängerer-Komponente,

   wobei die Polyisocyanat-Komponente a) mehr als 75 mol% Isophorondiisocyanat und die wenigstens eine polymere Polyolkomponente b) einen Polyester umfasst, und die hydrophilierende Komponente c) ein Sulfonat ist, und die aminofunktionelle Kettenverlängerer-Komponente d) mehr als 85 mol% Isophorondiamin umfasst.

2. Polyurethanharnstoff nach Anspruch 1, **dadurch gekennzeichnet, dass** die Polyisocyanat-Komponente a) ≥ 80 mol%, bevorzugt ≥ 85 mol%, weiter bevorzugt 95 mol% und besonders bevorzugt 100 mol% Isophorondiisocyanat umfasst.

3. Polyurethanharnstoff nach einem der Ansprüche 1 oder 2, **dadurch gekennzeichnet, dass** die polymere Polyolkomponente b) ein zahlenmittlere Molekulargewichte von ≥ 400 und ≤ 8000 g/mol und /oder eine OH-Funktionalitäten von 1,5 bis 6 aufweist.

4. Polyurethanharnstoff nach einem der Ansprüche 1 bis 3, **dadurch gekennzeichnet, dass** die polymere Polyolkomponente b) einen Polyester basierend auf Adipinsäure umfasst oder daraus besteht.

5. Polyurethanharnstoff nach Anspruch 4, **dadurch gekennzeichnet, dass** die aminofunktionelle Kettenverlängerer-Komponente d) ≥ 95 mol% und bevorzugt 100 mol% Isophorondiamin umfasst.

6. Verwendung eines Polyurethanharnstoffs erhältlich durch Umsetzung

   a) einer einzigen Polyisocyanat-Komponente,
   b) wenigstens einer polymeren Polyolkomponente,
   c) wenigstens einer hydrophilierenden Komponente und
   d) einer einzigen aminofunktionellen Kettenverlängerer-Komponente,

   wobei die Polyisocyanat-Komponente a) mehr als 75 mol% Isophorondiisocyanat und die aminofunktionelle Kettenverlängerer-Komponente d) mehr als 75 mol% Isophorondiamin umfasst,
   in der Kosmetik, bevorzugt im Bereich der Haarkosmetik, besonders bevorzugt im Bereich des Haarstylings.

7. Kosmetische Zusammensetzung, umfassend ein Lösungsmittelgemisch und einen Polyurethanharnstoff erhältlich durch Umsetzung

   a) einer einzigen Polyisocyanat-Komponente,
   b) wenigstens einer polymeren Polyolkomponente,
   c) wenigstens einer hydrophilierenden Komponente und
   d) einer einzigen aminofunktionellen Kettenverlängerer-Komponente,

   wobei die Polyisocyanat-Komponente a) mehr als 75 mol% Isophorondiisocyanat und die aminofunktionelle Kettenverlängerer-Komponente d) mehr als 75 mol% Isophorondiamin umfasst,
wobei das Lösungsmittelgemisch Ethanol und Wasser umfasst.

**8.** Zusammensetzung nach Anspruch 7, **dadurch gekennzeichnet, dass** das Lösungsmittelgemisch ≥ 10 Gew.-% und ≤ 98 Gew.-%, bevorzugt ≥ 20 Gew.-% und ≤ 98 Gew.-%, weiter bevorzugt ≥ 20 Gew.-% und ≤ 90 Gew.-% und besonders bevorzugt ≥ 20 Gew.-% und ≤ 80 Gew.-% Ethanol umfasst.

**9.** Zusammensetzung nach einem der Ansprüche 7 oder 8, **dadurch gekennzeichnet, dass** das Lösungsmittelgemisch aus Wasser und Ethanol besteht.

**10.** Zusammensetzung nach einem der Ansprüche 7 bis 9, **dadurch gekennzeichnet, dass** sie ≥ 0,1 Gew.-% und ≤ 30 Gew.-% , bevorzugt ≥ 0,1 Gew.-% und ≤ 20 Gew.-%, weiter bevorzugt ≥ 0,5 Gew.-% und ≤ 15 Gew.-% und besonders bevorzugt ≥ 0,5 Gew.-% und ≤ 10 Gew.-% des Polyurethanharnstoffs umfasst.

**11.** Zusammensetzung nach einem der Ansprüche 7 bis 10, **dadurch gekennzeichnet, dass** sie ≥ 10 Gew.-% und ≤ 98 Gew.-% , bevorzugt ≥ 20 Gew.-% und ≤ 98 Gew.-%, weiter bevorzugt ≥ 30 Gew.-% und ≤ 98 Gew.-% und besonders bevorzugt ≥ 40 Gew.-% und ≤ 98 Gew.-% des Lösungsmittelgemisches umfasst.

**12.** Verwendung einer kosmetischen Zusammensetzung nach einem der Ansprüche 7 bis 11 in der Kosmetik, bevorzugt im Bereich der Haarkosmetik, besonders bevorzugt im Bereich des Haarstylings.

**13.** Verfahren zum Formen von Frisuren, bei dem eine kosmetische Zusammensetzung nach einem der Ansprüche 7 bis 11 auf Haare aufgetragen wird.

## Claims

**1.** Polyurethane urea obtainable by reacting

a) a single polyisocyanate component,
b) at least one polymeric polyol component,
c) at least one hydrophilizing component and
d) a single amino-functional chain extender component,

where the polyisocyanate component a) comprises more than 75 mol% isophorone diisocyanate and the at least one polymeric polyol component b) comprises a polyester, and the hydrophilizing component c) is a sulfonate and the amino-functional chain extender component d) comprises more than 85 mol% isophoronediamine.

**2.** Polyurethane urea according to Claim 1, **characterized in that** the polyisocyanate component a) comprises ≥ 80 mol%, preferably ≥ 85 mol%, further preferably 95 mol% and particularly preferably 100 mol%, isophorone diisocyanate.

**3.** Polyurethane urea according to either of Claims 1 and 2, **characterized in that** the polymeric polyol component b) has a number-average molecular weights of ≥ 400 and ≤ 8000 g/mol and/or an OH functionalities of 1.5 to 6.

**4.** Polyurethane urea according to any one of Claims 1 to 3, **characterized in that** the polymeric polyol component b) comprises a polyester based on adipic acid, or consists thereof.

**5.** Polyurethane urea according to Claim 4, **characterized in that** the amino-functional chain extender component d) comprises ≥ 95 mol% and preferably 100 mol% isophoronediamine.

**6.** Use of a polyurethane urea obtainable by reacting

a) a single polyisocyanate component,
b) at least one polymeric polyol component,
c) at least one hydrophilizing component and
d) a single amino-functional chain extender component,

where the polyisocyanate component a) comprises more than 75 mol% isophorone diisocyanate and the amino-functional chain extender component d) comprises more than 75 mol% isophoronediamine,
in cosmetics, preferably in the field of hair cosmetics, particularly preferably in the field of hair styling.

**7.** Cosmetic composition comprising a solvent mixture and a polyurethane urea obtainable by reacting

a) a single polyisocyanate component,
b) at least one polymeric polyol component,
c) at least one hydrophilizing component and
d) a single amino-functional chain extender component,

where the polyisocyanate component a) comprises more than 75 mol% isophorone diisocyanate and the amino-functional chain extender component d) comprises more than 75 mol% isophoronediamine,
where the solvent mixture comprises ethanol and water.

**8.** Composition according to Claim 7, **characterized in that** the solvent mixture comprises ≥ 10% by weight and ≤ 98% by weight, preferably ≥ 20% by weight and ≤ 98% by weight, further preferably ≥ 20% by weight and ≤ 90% by weight and particularly preferably ≥ 20% by weight and ≤ 80% by weight, ethanol.

**9.** Composition according to either of Claims 7 and 8, **characterized in that** the solvent mixture consists of water and ethanol.

**10.** Composition according to any one of Claims 7 to 9, **characterized in that** it comprises ≥ 0.1% by weight and ≤ 30% by weight, preferably ≥ 0.1% by weight and ≤ 20% by weight, further preferably ≥ 0.5% by weight and ≤ 15% by weight and particularly preferably ≥ 0.5% by weight and ≤ 10% by weight, of the polyurethane urea.

**11.** Composition according to any one of Claims 7 to 10, **characterized in that** it comprises ≥ 10% by weight and ≤ 98% by weight, preferably ≥ 20% by weight and ≤ 98% by weight, further preferably ≥ 30% by weight and ≤ 98% by weight and particularly preferably ≥ 40% by weight and ≤ 98% by weight, of the solvent mixture.

**12.** Use of a cosmetic composition according to any one of Claims 7 to 11 in cosmetics, preferably in the field of hair cosmetics, particularly preferably in the field of hair styling.

**13.** Method for forming hairstyles, in which a cosmetic composition according to any one of Claims 7 to 11 is applied to hair.

## Revendications

**1.** Polyuréthane-urée pouvant être obtenu par mise en réaction de :

a) un composant polyisocyanate unique,
b) au moins un composant polyol polymère,
c) au moins un composant hydrophilisant, et
d) un composant allongeur de chaînes à fonction amino unique,

le composant polyisocyanate a) comprenant plus de 75 % en moles de diisocyanate d'isophorone et ledit au moins un composant polyol polymère b) comprenant un polyester, et le composant hydrophilisant c) étant un sulfonate, et le composant allongeur de chaînes à fonction amino d) comprenant plus de 85 % en moles d'isophorone-diamine.

**2.** Polyuréthane-urée selon la revendication 1, **caractérisé en ce que** le composant polyisocyanate a) comprend ≥ 80 % en moles, de préférence ≥ 85 % en moles, de manière davantage préférée 95 % en moles et de manière particulièrement préférée 100 % en moles de diisocyanate d'isophorone.

**3.** Polyuréthane-urée selon l'une quelconque des revendications 1 ou 2, **caractérisé en ce que** le composant polyol polymère b) présente un poids moléculaire moyen en nombre de ≥ 400 et ≤ 8 000 g/mol et/ou une fonctionnalité OH de 1,5 à 6.

**4.** Polyuréthane-urée selon l'une quelconque des revendications 1 à 3, **caractérisé en ce que** le composant polyol

polymère b) comprend un polyester à base d'acide adipique ou en est constitué.

5. Polyuréthane-urée selon la revendication 4, **caractérisé en ce que** le composant allongeur de chaînes à fonction amino d) comprend ≥ 95 % en moles et de préférence 100 % en moles d'isophorone-diamine.

6. Utilisation d'un polyuréthane-urée pouvant être obtenu par mise en réaction de :

   a) un composant polyisocyanate unique,
   b) au moins un composant polyol polymère,
   c) au moins un composant hydrophilisant, et
   d) un composant allongeur de chaînes à fonction amino unique,

   le composant polyisocyanate a) comprenant plus de 75 % en moles de diisocyanate d'isophorone, et le composant allongeur de chaînes à fonction amino d) comprenant plus de 75 % en moles d'isophorone-diamine,
   dans le domaine cosmétique, de préférence dans le domaine des cosmétiques capillaires, de manière particulièrement préférée dans le domaine des produits de coiffage des cheveux.

7. Composition cosmétique, comprenant un mélange de solvants et un polyuréthane-urée pouvant être obtenu par mise en réaction de :

   a) un composant polyisocyanate unique,
   b) au moins un composant polyol polymère,
   c) au moins un composant hydrophilisant, et
   d) un composant allongeur de chaînes à fonction amino unique,

   le composant polyisocyanate a) comprenant plus de 75 % en moles de diisocyanate d'isophorone, et le composant allongeur de chaînes à fonction amino d) comprenant plus de 75 % en moles d'isophorone-diamine,
   le mélange de solvants comprenant de l'éthanol et de l'eau.

8. Composition selon la revendication 7, **caractérisée en ce que** le mélange de solvants comprend ≥ 10 % en poids et ≤ 98 % en poids, de préférence ≥ 20 % en poids et ≤ 98 % en poids, de manière davantage préférée ≥ 20 % en poids et ≤ 90 % en poids, et de manière particulièrement préférée ≥ 20 % en poids et ≤ 80 % en poids d'éthanol.

9. Composition selon l'une quelconque des revendications 7 ou 8, **caractérisée en ce que** le mélange de solvants est constitué par de l'eau et de l'éthanol.

10. Composition selon l'une quelconque des revendications 7 à 9, **caractérisée en ce qu'**elle comprend ≥ 0,1 % en poids et ≤ 30 % en poids, de préférence ≥ 0,1 % en poids et ≤ 20 % en poids, de manière davantage préférée ≥ 0,5 % en poids et ≤ 15 % en poids, et de manière particulièrement préférée ≥ 0,5 % en poids et ≤ 10 % en poids du polyuréthane-urée.

11. Composition selon l'une quelconque des revendications 7 à 10, **caractérisée en ce qu'**elle comprend ≥ 10 % en poids et ≤ 98 % en poids, de préférence ≥ 20 % en poids et ≤ 98 % en poids, de manière davantage préférée ≥ 30 % en poids et ≤ 98 % en poids, et de manière particulièrement préférée ≥ 40 % en poids et ≤ 98 % en poids du mélange de solvants.

12. Utilisation d'une composition cosmétique selon l'une quelconque des revendications 7 à 11 dans le domaine cosmétique, de préférence dans le domaine des cosmétiques capillaires, de manière particulièrement préférée dans le domaine des produits de coiffage des cheveux.

13. Procédé de mise en forme de coiffures, selon lequel une composition cosmétique selon l'une quelconque des revendications 7 à 11 est appliquée sur des cheveux.

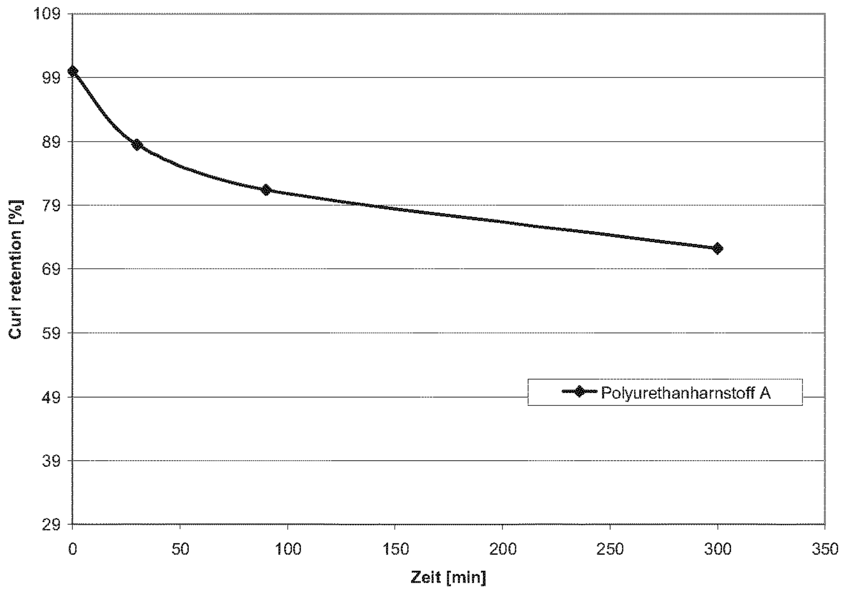
109
99
89
79
69
59
49
39
29
Curl retention [%]
0
50
100
150
200
250
300
350
Zeit [min]
Polyurethanharnstoff A

Figur 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009118105 A1
- DE 2446440 A
- US 3836537 A
- FR 1400366


### In der Beschreibung aufgeführte Nicht-Patentliteratur


- Ullmanns Encyclopädie der technischen Chemie. Verlag Chemie, vol. 19, 31-38